# EUROPEAN PATENT SPECIFICATION

(11) **EP 2 267 054 B1**
(45) Date of publication and mention of the grant of the patent: **22.08.2012**
(21) Application number: 10163202.4
(22) Date of filing: 19.05.2010
(51) Int. Cl.: C08G 63/08, G03G 9/08

(54) **Polyester synthesis**
Polyestersynthese
Synthèse de polyester

(30) Priority: 26.05.2009 US 471674
(43) Date of publication of application: 29.12.2010
(73) Proprietor: Xerox Corporation, Rochester, New York 14644 (US)
(72) Inventor: Wosnick, Jordan, Toronto Ontario M4S 2P5 (CA); Moffat, Karen A., Brantford Ontario N3R 7P8 (CA)
(74) Representative: Grünecker, Kinkeldey, Stockmair & Schwanhäusser

(56) References cited:
- JP-A- 2007 025 656
- JP-A- 2007 033 716
- US-A1- 2009 104 276

## Description

### BACKGROUND

The present disclosure is generally directed to polyester synthesis processes and, more specifically, to processes for the synthesis of polyester resins which may be utilized in the formation of emulsion aggregation toners.

Electrophotographic printing utilizes toner particles which may be produced by a variety of processes. One such process includes an emulsion aggregation ("EA") process that forms toner particles in which surfactants are used in forming a latex emulsion. See, for example, U.S. Patent No. 6,120,967, as one example of such a process.

Furthermore, Japan Patent No. 2007 033716 discloses toner particles with a core-shell structure, wherein the core contains polylactone. The toner also comprises a colorant and a binder resin. The Japan Patent No. 2007025656 discloses a toner comprising a polyester resin, which is obtained by a ring opening polymerization of a cyclic ester. The U.S. Patent No. 2009/0104276 A1 discloses a semi-crystalline copolyester composition comprising the reaction product of a polycondensation polyester and a lactone.

Combinations of amorphous and crystalline polyesters may be used in the EA process. This resin combination provides toners with high gloss and relatively low-melting point characteristics (sometimes referred to as low-melt, ultra low melt, or ULM), which allows for more energy efficient and faster printing. The choice of crystalline polymer may be important as poor crystalline-amorphous polymer combinations may result in toners that either do not show low-melt behavior or exhibit unacceptable heat cohesion properties.

Control of the distribution of the crystalline component within a polyester EA toner particle may be important in realizing optimal toner performance, especially in the area of charging, where crystalline polyesters on the particle surface can lead to poor charge. For example, EA ULM toners have been developed which use an amorphous polyester shell to limit the migration of crystalline polyester to the toner particle surface. The crystalline component may be sequestered in the interior of core-shell nanoparticles, surrounded by an amorphous resin shell. Molecule-level confinement may thus prevent the crystalline material from migrating to the toner particle surface, thereby providing desirable charging characteristics.

There is a continual need for improving polyester resins synthesis, as well as the use of polyesters in the formation ofEA ULM toners.

### SUMMARY

The present disclosure provides processes for the production of block copolymer polyester resins suitable for use in manufacturing toners, as well as compositions including such copolymers. In embodiments, a process of the present disclosure may include contacting a first component with a catalyst, optionally in solution; polymerizing the first component to form a first block of a copolyester resin; contacting the first block, optionally in solution, with a second component; polymerizing the second component to form a second block of the copolyester resin linked to the first block; and recovering the resulting copolyester resin including a crystalline block and an amorphous block; wherein one of the first and second components is at least one precursor of an amorphous block comprising a lactone, a cyclic anhydrid, a cyclic carbonateor a combination thereof, and the other of the first and second components is at least one precursor of a crystalline block comprising a lactone, a cyclic anhydrid, a cyclic carbonate or a combination thereof

In other embodiments, a process of the present disclosure may include contacting at least one precursor of an amorphous block including a lactone such as 4-tert-butylcaprolactone, 4-phenylcaprolactone, 4-methylcaprolactone, 3,5-dimethylcaprolactone, 3-isochromanone, 4-(sulfonatophenyl)caprolactone, lactide, glycolide, cyclic anhydrides, cyclic carbonates, and combinations thereof with a catalyst, optionally in solution; polymerizing the at least one precursor of the amorphous block to form an amorphous block; contacting the amorphous block with at least one precursor of a crystalline block including a lactone such as caprolactone, 1,4-dioxan-2-one, valerolactone and combinations thereof, optionally with a catalyst, optionally in solution; polymerizing the at least one precursor of the crystalline block to form a crystalline block linked to the amorphous block; and recovering a copolyester resin including the amorphous block and the crystalline block.

Compositions including these copolymers are also provided. In embodiments, a composition of the present disclosure may include a copolyester resin including core-shell particles including a crystalline block obtained from the polymerization of a lactone such as caprolactone, 1,4-dioxan-2-one, valerolactone, and combinations thereof as the core, in combination with an amorphous block obtained from the polymerization of a lactone such as 4-tert-butylcaprolactone, 4-phenylcaprolactone, 4-methylcaprolactone, 3,5-dimethylcaprolactone, 3-isochromanone, lactide, glycolide, 4-(sulfonatophenyl)caprolactone, and combinations thereof as the shell; at least one colorant; and an optional wax, and an optional surfactant, wherein the crystalline block of the copolyester resin is present in an amount of from about 1 to about 90 percent by weight of the copolyester resin and possesses a melting temperature of from about 20°C to about 200°C, and the amorphous block of the copolyester resin is present in an amount of from about 10 to about 99 percent by weight of the copolyester resin and possesses a glass transition temperature of from about 0°C to about 200°C.

### DETAILED DESCRIPTION

The present disclosure relates to polymerization processes for the production of resins suitable for use in the formation of toners. In embodiments, processes of the present disclosure may be utilized to produce block copolymers including distinct crystalline polyester blocks and distinct amorphous polyester blocks. These copolymers may self-assemble in water or a similar media to form nanoparticles suitable for forming toner compositions. In embodiments, the nanoparticles may possess a core-shell configuration, with the crystalline block forming the core and the amorphous block forming the shell.

In embodiments, core-shell polyester nanoparticles may be formed through the ring-opening polymerization (ROP) of lactones. ROP catalysts and/or initiators may be utilized to facilitate the formation of these block co-polymers. By suitable choice of lactone monomers and block sizes, polyesters containing one or more amorphous blocks linked to one or more crystalline blocks may be prepared.

In embodiments, other starting materials besides lactones may be utilized to form the desired block copolymers. For example, the ring-opening co-polymerization of cyclic anhydrides, cyclic carbonates , and combinations thereof, may also be used, optionally in combination with lactones, to form copolymers of the present disclosure.

### Resins

Any monomer or starting material suitable for preparing a resin for use in a toner may be utilized. In embodiments of the present disclosure, the resin may be a block copolymer including at least one amorphous polyester block and at least one separate crystalline polyester block formed by the ring-opening polymerization of a lactone, cyclic anhydride, and/or cyclic carbonate. In embodiments, the polyester copolymers may be obtained by the ring-opening polymerization of two or more lactones, cyclic anhydrides and/or cyclic carbonates. The starting materials may be selected so that at least one of the starting monomers forms a crystalline block, with at least one other monomer forming an amorphous block in the resulting polyester copolymer. For example, in embodiments, a crystalline block may be formed by the ROP of lactones, including, but not limited to, caprolactone, valerolactone, 1,4-dioxan-2-one,glycolide, combinations thereof, and the like. In embodiments, both a caprolactone and 1,4-dioxan-2-one may be utilized to form the crystalline block. In such a case, the resulting copolymer may be a poly(caprolactone-block-1,4-dioxan-2-one). Gel permeation chromatography (GPC), ¹³C NMR, and differential scanning calorimetry may be utilized to characterize the resulting copolymer. Poly(caprolactone-block-1,4-dioxan-2-one) may exhibit a single GPC peak but two distinct melting points (55°C and 103°C), corresponding to the melting points of its constituent homopolymeric units.

In other embodiments, the crystalline component of the block copolymer may be obtained from the ring-opening polymerization of cyclic anhydrides, cyclic carbonates, combinations thereof, and the like. Suitable cyclic anhydrides include, but are not limited to, aliphatic dicarboxylic anhydrides such as succinic anhydride, glutaric anhydride, maleic anhydride, and combinations thereof. Suitable cyclic carbonates include, but are not limited to, trimethylene carbonate, carboxy-trimethylene carbonate and esters thereof, combinations thereof, and the like.

As noted above, in embodiments, the starting materials, such as lactones, may be chosen such that one of the formed blocks is amorphous in nature, and the other is crystalline. Examples of lactones that produce amorphous polyesters in ring-opening polymerizations include 4-tert-butylcaprolactone, 4-phenylcaprolactone, 4-methylcaprolactone and its isomers, 3,5-dimethylcaprolactone and its isomers, 3-isochromanone, lactide, glycolide, 4-(sulfonatophenyl)caprolactone, cyclic anhydrides such as cyclopentane-1,2-dicarboxylic anhydride, cyclohexene-1,2-dicarboxylic anhydride and 2,3-bicyclo[2,2,2]octadicarboxylic anhydride. Examples of aromatic dicarboxylic anhydrides include phthalic anhydride, naphthalene-dicarboxylic anhydride, pyridine-dicarboxylic anhydride and thiophene-dicarboxylic anhydride, cyclic carbonates, combinations thereof, and the like.

### Catalysts

In embodiments, the ring-opening polymerization described above may take place in the presence of a catalyst. Catalysts which may be utilized to form the block copolymers of the present disclosure include, but are not limited to, aluminum isopropoxide (Al(OiPr)₃), yttrium isopropoxide (Y(OiPr)₃), stannous octoate (Sn(Oct)₂), scandium trifluoromethane sulfonate (Sc(OTf)₃), stannous trifluoromethane sulfonate (Sn(OTf)₂), combinations thereof, and the like. In embodiments, these catalysts may mediate ROP by quasi-living polymerization mechanisms, making them suitable for the formation of block co-polymers with chemically distinct blocks.

In other embodiments, copolymers of the present disclosure may be produced by enzymatic polymerization. Suitable enzymes to form the polyesters include *Pseudomonas* family lipases, such as lipases from *Pseudomonas aeruginosa* (lipase PA), *Pseudomonas cepacia* (lipase PC), *Pseudomonas fluorescens* (lipase PF), as well as lipases from *Aspegillus niger* (lipase A), *Candida antarctica* (lipase CA or lipase B), *Candida cylindracea* (lipase CC), *Klebsiella oxytoca* (lipase K), *Mucor meihei* (lipase MM), cutinases such as the cutinase from *Humicola insolens*, combinations thereof, and the like.

The lipases or other enzymes may be obtained from the above organisms utilizing methods within the purview of those skilled in the art. In embodiments, the organisms may be grown in incubation vessels and fed with nutrients and sugars (e.g., glucose). By selecting the right growth and treatment conditions, lipases may be obtained from the organisms. In other embodiments, the lipases or other enzymes may be obtained from commercial sources, for example, Fluka BioChemika, Novozymes, and/or Sigma Aldrich. In embodiments, the catalyst may be immobilized on a support member such as porous polymer beads, acrylic resins, cross-linked polystyrene, or any other suitable polymeric and/or ceramic supports within the purview of those skilled in the art.

The catalysts utilized to form the polyester copolymers of the present disclosure should be able to operate at temperatures of from about 10° C to about 100° C, in embodiments from about 20° C to about 90° C, in other embodiments from about 45° C to about 75° C, although temperatures outside these ranges can be utilized.

Same or different catalysts may be utilized to form the at least one crystalline block and the at least one amorphous block.

In embodiments, the amount of catalyst utilized to catalyze a reaction may be from about 0.01 % by weight to about 10 % by weight based on the starting materials used to generate the copolymer polyester resin, in embodiments from about 0.1 % by weight to about 6 % by weight based on the starting materials used to generate the copolymer polyester resin, although amounts outside these ranges can be utilized.

### Reaction Conditions

The starting materials utilized to form the copolymer polyester resin of the present disclosure, for example the lactones, cyclic anhydrides, and/or cyclic carbonates described above, may be combined with the above catalysts and a polyester may be formed. The polymerization may be performed in the presence or absence of solvents.

In the polymerization process, the reactants may be added to a suitable reactor, such as a mixing vessel. The appropriate amount of starting materials may be optionally dissolved in a solvent, a catalyst may be added to the solution, and a polyester copolymer formed which may then be used in the production of a toner. In other embodiments, the starting materials may be combined with the catalysts without solvent and a polyester formed. Where utilized, suitable solvents include, but are not limited to, water and/or organic solvents including toluene, benzene, xylene, tetrahydrofuran, dichloromethane, combinations thereof, and the like. The starting material(s) and catalyst(s) utilized to form the crystalline block may be in solution, the starting material(s) and catalyst(s) utilized to form the amorphous block may be in solution, or both starting materials may be in the same or separate solutions.

Formation of the blocks may be simultaneous or sequential. For example, in some embodiments, a catalyst and starting material utilized to form one of the blocks may be in solution, the block allowed to form, followed by addition of the starting material utilized to form the second block, optionally in solution. In such a case, the starting material for one of the blocks, sometimes referred to herein as a first component, may be contacted with a catalyst, optionally in solution, followed by polymerizing the first component to form a first block of a copolyester resin. The first block in solution may then be contacting with the starting material utilized to form a second block of the copolyester, sometimes referred to herein as a second component, followed by polymerizing the second component to form a second block of the copolyester resin linked to the first block. The resulting copolyester resin, which includes a crystalline block and an amorphous block, may then be recovered.

Thus, for example, in embodiments, starting material(s) for forming a crystalline block, sometimes referred to herein as the precursor(s) of the crystalline block, may first be combined with a catalyst, optionally in a solution, to form the crystalline block, followed by the addition of the starting material(s) for the formation of the amorphous block, sometimes referred to herein as the precursor(s) of the amorphous block, optionally in solution, and optionally with additional catalyst, and the subsequent formation of the amorphous block. In other embodiments, the order of formation of the blocks may be reversed, i.e., formation of an amorphous block by combining the starting materials/precursors with a catalyst, optionally in a solution, followed by formation of a crystalline block by the addition of the appropriate starting materials/precursors, optionally in solution, and optionally with additional catalyst.

Where the starting materials are in solution, the starting materials may be at a concentration of from about 10% by weight to about 90% by weight, in embodiments from about 30% by weight to about 60% by weight, although amounts outside these ranges can be used.

As noted above, in embodiments, multiple lactones, cyclic anhydrides and/or cyclic carbonates may be utilized in forming a crystalline block in the resulting copolymer, and at least one other lactone may be utilized in forming an amorphous block in the resulting copolymer. Suitable monomers include lactones such as 4-tert-butylcaprolactone, 4-phenylcaprolactone, 4-methylcaprolactone and its isomers, 3,5-dimethylcaprolactone and its isomers, 3-isochromanone, lactide, glycolide, 4-(sulfonatophenyl)caprolactone, cyclic anhydrides such as cyclopentane-1,2-dicarboxylic anhydride, cyclohexene-1,2-dicarboxylic anhydride and 2,3-bicyclo[2,2,2]octadicarboxylic anhydride, phthalic anhydride, naphthalene-dicarboxylic anhydride, pyridine-dicarboxylic anhydride and thiophene-dicarboxylic anhydride, cyclic carbonates,

combinations thereof, and the like.

The time for the reaction may depend upon the type and amount of starting materials utilized, the amount of catalyst utilized, the temperature of the reaction, and the like. In embodiments, the reaction mixture may be mixed for from about 1 minute to about 72 hours, in embodiments from about 4 hours to about 24 hours (although times outside these ranges can be used), while keeping the temperature within the operational range of the catalyst being used, in embodiments from about 10° C to about 100° C, in embodiments from about 20° C to about 90° C, in other embodiments from about 45° C to about 75° C, although temperatures outside these ranges can be used.

Those skilled in the art will recognize that optimization of reaction conditions, temperature, and catalyst loading can be varied to generate polyesters of various molecular weights, and that structurally related starting materials may be polymerized using comparable techniques.

The resins thus produced may include crystalline blocks having a glass transition temperature (Tg) of from about -60°C to about 200°C, in embodiments from about -50°C to about 20°C, and a melting temperature of from about 20°C to about 200°C, in embodiments form about 55°C to about 95°C. The resins thus produced may also include amorphous blocks having a glass transition temperature (Tg) of from about 0°C to about 200°C, in embodiments from about 5°C to about 60°C.

The copolymers may have a number average molecular weight (Mₙ), as measured by gel permeation chromatography (GPC) of, for example, from about 2,000 to about 200,000, in embodiments from about 10,000 to about 100,000, and a weight average molecular weight (M_{w}) of, for example, from about 2,000 to about 200,000, in embodiments from about 10,000 to about 100,000, as determined by Gel Permeation Chromatography using polystyrene standards. The molecular weight distribution (M_{w}/Mₙ) of the copolymer may be, for example, from about 1.01 to about 4.0, in embodiments from about 1.1 to about 2.0.

The resulting copolymer may possess crystalline blocks in amounts of from about 1 to about 90 percent by weight of the block copolymer, in embodiments from about 5 to about 30 percent by weight of the block copolymer, and amorphous blocks in amounts of from about 10 to about 99 percent by weight of the block copolymer, in embodiments from about 70 to about 95 percent by weight of the block copolymer.

The weight of the resulting polymers may depend on the starting materials, reaction conditions, and the catalyst being used. Higher temperatures, in embodiments about 60 °C or above, and longer reaction times of about forty eight (48) or more hours, may yield polymers with higher molecular weights.

In embodiments, the final copolymer polyester may be utilized to form toner particles. In embodiments, if the particle size of the polyester is too large, the particles may be subjected to homogenizing or sonication to further disperse the nanoparticles and break apart any agglomerates or loosely bound particles. Where utilized, a homogenizer, (that is, a high shear device), may operate at a rate of from about 6,000 rpm to about 10,000 rpm, in embodiments from about 7,000 rpm to about 9,750 rpm, for a period of time of from about 0.5 minutes to about 60 minutes, in embodiments from about 5 minute to about 30 minutes, although speeds and times outside these ranges may be utilized.

In embodiments, a suitable choice of monomer combinations and block lengths produces polymers that spontaneously self-assemble into core-shell nanoparticles when placed in water or a similar media such as mixtures of water and alcohol, water and tetrahydrofuran, and the like. For example, a tri-block co-polymer such as poly[caprolactone-block-4-phenylcaprolactone-block-4-(sulfonatophenyl)caprolactone], when dispersed in water, may organize into core-shell nanoparticles with an inner core of polycaprolactone, an intermediate poly(4-phenylcaprolactone) layer, and a water-stabilizing poly(4-(sulfonatophenyl)caprolactone) shell. This particle can then be incorporated into toner with other standard toner ingredients using an emulsion-aggregation process.

In other embodiments, cross-linkable vinylic groups may be present at one or both ends of the copolymer polyester of the present disclosure. These groups may be introduced through, for example, alcohol intiation of ROP catalyzed by Sc(OTf)₃, as described by Okada and co-workers (Macromolecules 2000, 33, pp. 1497-1499) and by Hedrick and co-workers (J. Polym. Sci. A, 2000, 38, pp. 2067-2074). For example, the ROP of lactide, initiated by 2-hydroxyethyl methacrylate (HEMA) and catalyzed by Sc(OTf)₃, followed by addition of caprolactone, would result in a block co-polymer terminated by a single methacrylate group. Other suitable initiators include, but are not limited to, 3-hydroxypropyl methacrylate, 2-hydroxyethyl acrylate, 3-hydroxypropyl acrylate, glycerol mono-methacrylate, combinations thereof, and the like.

Core-shell particles formed from these block co-polymers could thus have components forming the shell, including these vinylic groups, cross-linked through radical polymerization of the methacrylate groups or other vinylic groups using standard methods, providing a method to increase the shell rigidity and/or glass transition temperature of the particles.

Similarly, in other embodiments, core-shell particles formed from these block co-polymers could have components forming the core including these vinylic groups cross-linked through radical polymerization of the methacrylate groups or other vinylic groups therein using standard methods, altering properties such as particle stability, melt viscosity and/or glass transition temperature of the particles.

The crystalline block of the copolymer resin may be present, for example, in an amount of from about 5 to about 30 percent by weight of the toner components, in embodiments from about 10 to about 20 percent by weight of the toner components. The amorphous block of the copolymer resin may be present, for example, in an amount of from about 5 to about 75 percent by weight of the toner components, in embodiments from about 20 to about 60 percent by weight of the toner components.

### Toner

The copolyester resin described above may then be utilized to form toner compositions. Toner compositions of the present disclosure may also include optional colorants, waxes, and other additives. Toners may be formed utilizing any method within the purview of those skilled in the art.

### Surfactants

In embodiments, colorants, waxes, and other additives utilized to form toner compositions may be in dispersions including surfactants. Moreover, toner particles may be formed by emulsion aggregation methods where the copolymer resin described above and other components of the toner are placed in one or more surfactants, an emulsion is formed, toner particles are aggregated, coalesced, optionally washed and dried, and recovered.

One, two, or more surfactants may be utilized. The surfactants may be selected from ionic surfactants and nonionic surfactants. Anionic surfactants and cationic surfactants are encompassed by the term "ionic surfactants." In embodiments, the surfactant may be utilized so that it is present in an amount of from about 0.01% to about 5% by weight of the toner composition, for example from about 0.75% to about 4% by weight of the toner composition, in embodiments from about 1% to about 3% by weight of the toner composition, although amounts outside these ranges may be utilized.

Examples of nonionic surfactants that can be utilized include, for example, polyacrylic acid, methalose, methyl cellulose, ethyl cellulose, propyl cellulose, hydroxy ethyl cellulose, carboxy methyl cellulose, polyoxyethylene cetyl ether, polyoxyethylene lauryl ether, polyoxyethylene octyl ether, polyoxyethylene octylphenyl ether, polyoxyethylene oleyl ether, polyoxyethylene sorbitan monolaurate, polyoxyethylene stearyl ether, polyoxyethylene nonylphenyl ether, dialkylphenoxy poly(ethyleneoxy) ethanol, available from Rhone-Poulenc as IGEPAL CA-210™, IGEPAL CA-520™, IGEPAL CA-720™, IGEPAL CO-890™, IGEPAL CO-720™, IGEPAL CO-290™, IGEPAL CA-210™, ANTAROX 890™ and ANTAROX 897™. Other examples of suitable nonionic surfactants include a block copolymer of polyethylene oxide and polypropylene oxide, including those commercially available as SYNPERONIC PE/F, in embodiments SYNPERONIC PE/F 108.

Anionic surfactants which may be utilized include sulfates and sulfonates, sodium dodecylsulfate (SDS), sodium dodecylbenzene sulfonate, sodium dodecylnaphthalene sulfate, dialkyl benzenealkyl sulfates and sulfonates, acids such as abitic acid available from Aldrich, NEOGEN R™, NEOGEN SC™ obtained from Daiichi Kogyo Seiyaku, combinations thereof, and the like. Other suitable anionic surfactants include, in embodiments, DOWFAX™ 2A1, an alkyldiphenyloxide disulfonate from The Dow Chemical Company, and/or TAYCA POWER BN2060 from Tayca Corporation (Japan), which are branched sodium dodecyl benzene sulfonates. Combinations of these surfactants and any of the foregoing anionic surfactants may be utilized in embodiments.

Examples of the cationic surfactants, which are usually positively charged, include, for example, alkylbenzyl dimethyl ammonium chloride, dialkyl benzenealkyl ammonium chloride, lauryl trimethyl ammonium chloride, alkylbenzyl methyl ammonium chloride, alkyl benzyl dimethyl ammonium bromide, benzalkonium chloride, cetyl pyridinium bromide, C₁₂, C₁₅, C₁₇ trimethyl ammonium bromides, halide salts of quaternized polyoxyethylalkylamines, dodecylbenzyl triethyl ammonium chloride, MIRAPOL™ and ALKAQUAT™, available from Alkaril Chemical Company, SANIZOL™ (benzalkonium chloride), available from Kao Chemicals, and the like, and mixtures thereof.

### Colorants

As the colorant to be added, various known suitable colorants, such as dyes, pigments, mixtures of dyes, mixtures of pigments, mixtures of dyes and pigments, and the like, may be included in the toner. The colorant may be included in the toner in an amount of, for example, about 0.1 to about 35 percent by weight of the toner, or from about 1 to about 15 weight percent of the toner, or from about 3 to about 10 percent by weight of the toner, although amounts outside these ranges may be utilized.

As examples of suitable colorants, mention may be made of carbon black like REGAL 330^{®}; magnetites, such as Mobay magnetites MO8029™, MO8060™; Columbian magnetites; MAPICO BLACKS™ and surface treated magnetites; Pfizer magnetites CB4799™, CB5300™, CB5600™, MCX6369™; Bayer magnetites, BAYFERROX 8600™, 8610™; Northern Pigments magnetites, NP-604™, NP-608™; Magnox magnetites TMB-100™, or TMB-104™; and the like. As colored pigments, there can be selected cyan, magenta, yellow, red, green, brown, blue or mixtures thereof. Generally, cyan, magenta, or yellow pigments or dyes, or mixtures thereof, are used. The pigment or pigments are generally used as water based pigment dispersions.

Specific examples of pigments include SUNSPERSE 6000, FLEXIVERSE and AQUATONE water based pigment dispersions from SUN Chemicals, HELIOGEN BLUE L6900™, D6840™, D7080™, D7020™, PYLAM OIL BLUE™, PYLAM OIL YELLOW™, PIGMENT BLUE 1™ available from Paul Uhlich & Company, Inc., PIGMENT VIOLET 1™, PIGMENT RED 48™, LEMON CHROME YELLOW DCC 1026™, E.D. TOLUIDINE RED™ and BON RED C™ available from Dominion Color Corporation, Ltd., Toronto, Ontario, NOVAPERM YELLOW FGL™, HOSTAPERM PINK E™ from Hoechst, and CINQUASIA MAGENTA™ available from E.I. DuPont de Nemours & Company, and the like. Generally, colorants that can be selected are black, cyan, magenta, or yellow, and mixtures thereof. Examples of magentas are 2,9-dimethyl-substituted quinacridone and anthraquinone dye identified in the Color Index as CI 60710, CI Dispersed Red 15, diazo dye identified in the Color Index as CI 26050, CI Solvent Red 19, and the like. Illustrative examples of cyans include copper tetra(octadecyl sulfonamido) phthalocyanine, x-copper phthalocyanine pigment listed in the Color Index as CI 74160, CI Pigment Blue, Pigment Blue 15:3, and Anthrathrene Blue, identified in the Color Index as CI 69810, Special Blue X-2137, and the like. Illustrative examples of yellows are diarylide yellow 3,3-dichlorobenzidene acetoacetanilides, a monoazo pigment identified in the Color Index as CI 12700, CI Solvent Yellow 16, a nitrophenyl amine sulfonamide identified in the Color Index as Foron Yellow SE/GLN, CI Dispersed Yellow 33 2,5-dimethoxy-4-sulfonanilide phenylazo-4'-chloro-2,5-dimethoxy acetoacetanilide, and Permanent Yellow FGL. Colored magnetites, such as mixtures of MAPICO BLACK™, and cyan components may also be selected as colorants. Other known colorants can be selected, such as Levanyl Black A-SF (Miles, Bayer) and Sunsperse Carbon Black LHD 9303 (Sun Chemicals), and colored dyes such as Neopen Blue (BASF), Sudan Blue OS (BASF), PV Fast Blue B2G01 (American Hoechst), Sunsperse Blue BHD 6000 (Sun Chemicals), Irgalite Blue BCA (Ciba-Geigy), Paliogen Blue 6470 (BASF), Sudan III (Matheson, Coleman, Bell), Sudan II (Matheson, Coleman, Bell), Sudan IV (Matheson, Coleman, Bell), Sudan Orange G (Aldrich), Sudan Orange 220 (BASF), Paliogen Orange 3040 (BASF), Ortho Orange OR 2673 (Paul Uhlich), Paliogen Yellow 152, 1560 (BASF), Lithol Fast Yellow 0991K (BASF), Paliotol Yellow 1840 (BASF), Neopen Yellow (BASF), Novoperm Yellow FG 1 (Hoechst), Permanent Yellow YE 0305 (Paul Uhlich), Lumogen Yellow D0790 (BASF), Sunsperse Yellow YHD 6001 (Sun Chemicals), Suco-Gelb L1250 (BASF), Suco-Yellow D1355 (BASF), Hostaperm Pink E (American Hoechst), Fanal Pink D4830 (BASF), Cinquasia Magenta (DuPont), Lithol Scarlet D3700 (BASF), Toluidine Red (Aldrich), Scarlet for Thermoplast NSD PS PA (Ugine Kuhlmann of Canada), E.D. Toluidine Red (Aldrich), Lithol Rubine Toner (Paul Uhlich), Lithol Scarlet 4440 (BASF), Bon Red C (Dominion Color Company), Royal Brilliant Red RD-8192 (Paul Uhlich), Oracet Pink RF (Ciba-Geigy), Paliogen Red 3871K (BASF), Paliogen Red 3340 (BASF), Lithol Fast Scarlet L4300 (BASF), combinations of the foregoing, and the like. Wax

Optionally, a wax may also be combined with the resin and a colorant in forming toner particles. When included, the wax may be present in an amount of, for example, from about 1 weight percent to about 25 weight percent of the toner particles, in embodiments from about 5 weight percent to about 20 weight percent of the toner particles, although amounts outside these ranges may be utilized.

Waxes that may be selected include waxes having, for example, a weight average molecular weight of from about 500 to about 20,000, in embodiments from about 1,000 to about 10,000, although weights outside these ranges may be utilized. Waxes that may be used include, for example, polyolefins such as polyethylene, polypropylene, and polybutene waxes such as commercially available from Allied Chemical and Petrolite Corporation, for example POLYWAX™ polyethylene waxes from Baker Petrolite, wax emulsions available from Michaelman, Inc. and the Daniels Products Company, EPOLENE N-15™ commercially available from Eastman Chemical Products, Inc., and VISCOL 550-P™, a low weight average molecular weight polypropylene available from Sanyo Kasei K. K.; plant-based waxes, such as carnauba wax, rice wax, candelilla wax, sumacs wax, and jojoba oil; animal-based waxes, such as beeswax; mineral-based waxes and petroleum-based waxes, such as montan wax, ozokerite, ceresin, paraffin wax, microcrystalline wax, and Fischer-Tropsch wax; ester waxes obtained from higher fatty acid and higher alcohol, such as stearyl stearate and behenyl behenate; ester waxes obtained from higher fatty acid and monovalent or multivalent lower alcohol, such as butyl stearate, propyl oleate, glyceride monostearate, glyceride distearate, and pentaerythritol tetra behenate; ester waxes obtained from higher fatty acid and multivalent alcohol multimers, such as diethyleneglycol monostearate, dipropyleneglycol distearate, diglyceryl distearate, and triglyceryl tetrastearate; sorbitan higher fatty acid ester waxes, such as sorbitan monostearate, and cholesterol higher fatty acid ester waxes, such as cholesteryl stearate. Examples of functionalized waxes that may be used include, for example, amines, amides, for example AQUA SUPERSLIP 6550™, SUPERSLIP 6530™ available from Micro Powder Inc., fluorinated waxes, for example POLYFLUO 190™, POLYFLUO 200™, POLYSILK 19™, POLYSILK 14™ available from Micro Powder Inc., mixed fluorinated, amide waxes, for example MICROSPERSION 19™ also available from Micro Powder Inc., imides, esters, quaternary amines, carboxylic acids or acrylic polymer emulsion, for example JONCRYL 74™, 89™, 130™, 537™, and 538™, all available from SC Johnson Wax, and chlorinated polypropylenes and polyethylenes available from Allied Chemical and Petrolite Corporation and SC Johnson wax. Mixtures and combinations of the foregoing waxes may also be used in embodiments. Waxes may be included as, for example, fuser roll release agents.

### Toner Preparation

The toner particles may be prepared by any method within the purview of one skilled in the art. Although embodiments relating to toner particle production are described below with respect to emulsion-aggregation processes, any suitable method of preparing toner particles may be used, including chemical processes, such as suspension and encapsulation processes disclosed in U.S. Patent Nos. 5,290,654 and 5,302,486. In embodiments, toner compositions and toner particles may be prepared by aggregation and coalescence processes in which small-size resin particles are aggregated to the appropriate toner particle size and then coalesced to achieve the final toner particle shape and morphology.

In embodiments, toner compositions may be prepared by emulsion-aggregation processes, such as a process that includes aggregating a mixture of an optional colorant, an optional wax and any other desired or required additives, and emulsions including the copolymer resins described above, optionally in surfactants as described above, and then coalescing the aggregate mixture. A mixture may be prepared by adding a colorant and optionally a wax or other materials, which may also be optionally in a dispersion(s) including a surfactant, to the emulsion, which may be a mixture of two or more emulsions containing the resin. The pH of the resulting mixture may be adjusted by an acid such as, for example, acetic acid, nitric acid or the like. In embodiments, the pH of the mixture may be adjusted to from about 4 to about 5, although a pH outside this range may be utilized. Additionally, in embodiments, the mixture may be homogenized. If the mixture is homogenized, homogenization may be accomplished by mixing at about 600 to about 4,000 revolutions per minute, although speeds outside this range may be utilized. Homogenization may be accomplished by any suitable means, including, for example, an IKA ULTRA TURRAX T50 probe homogenizer.

Following the preparation of the above mixture, an aggregating agent may be added to the mixture. Any suitable aggregating agent may be utilized to form a toner. Suitable aggregating agents include, for example, aqueous solutions of a divalent cation or a multivalent cation material. The aggregating agent may be, for example, polyaluminum halides such as polyaluminum chloride (PAC), or the corresponding bromide, fluoride, or iodide, polyaluminum silicates such as polyaluminum sulfosilicate (PASS), and water soluble metal salts including aluminum chloride, aluminum nitrite, aluminum sulfate, potassium aluminum sulfate, calcium acetate, calcium chloride, calcium nitrite, calcium oxylate, calcium sulfate, magnesium acetate, magnesium nitrate, magnesium sulfate, zinc acetate, zinc nitrate, zinc sulfate, zinc chloride, zinc bromide, magnesium bromide, copper chloride, copper sulfate, and combinations thereof. In embodiments, the aggregating agent may be added to the mixture at a temperature that is below the glass transition temperature (Tg) of the resin.

The aggregating agent may be added to the mixture utilized to form a toner in an amount of, for example, from about 0.1% to about 8% by weight, in embodiments from about 0.2% to about 5% by weight, in other embodiments from about 0.5% to about 5% by weight, of the resin in the mixture, although amounts outside these ranges may be utilized. This provides a sufficient amount of agent for aggregation.

In order to control aggregation and coalescence of the particles, in embodiments the aggregating agent may be metered into the mixture over time. For example, the agent may be metered into the mixture over a period of from about 5 to about 240 minutes, in embodiments from about 30 to about 200 minutes, although more or less time may be used as desired or required. The addition of the agent may also be done while the mixture is maintained under stirred conditions, in embodiments from about 50 rpm to about 1,000 rpm, in other embodiments from about 100 rpm to about 500 rpm (although speeds outside these ranges may be utilized), and at a temperature that is below the glass transition temperature of the resin as discussed above, in embodiments from about 30 °C to about 90 °C, in embodiments from about 35°C to about 70 °C, although temperatures outside these ranges may be utilized.

The particles may be permitted to aggregate and/or coalesce until a predetermined desired particle size is obtained. A predetermined desired size refers to the desired particle size to be obtained as determined prior to formation, and the particle size being monitored during the growth process until such particle size is reached. Samples may be taken during the growth process and analyzed, for example with a Coulter Counter, for average particle size. The aggregation/coalescence thus may proceed by maintaining the elevated temperature, or slowly raising the temperature to, for example, from about 40°C to about 100°C (although temperatures outside this range may be utilized), and holding the mixture at this temperature for a time from about 0.5 hours to about 6 hours, in embodiments from about hour 1 to about 5 hours (although times outside these ranges maybe utilized), while maintaining stirring, to provide the aggregated particles. Once the predetermined desired particle size is reached, then the growth process is halted. In embodiments, the predetermined desired particle size is within the toner particle size ranges mentioned above.

The growth and shaping of the particles following addition of the aggregation agent may be accomplished under any suitable conditions. For example, the growth and shaping may be conducted under conditions in which aggregation occurs separate from coalescence. For separate aggregation and coalescence stages, the aggregation process may be conducted under shearing conditions at an elevated temperature, for example of from about 40°C to about 90°C, in embodiments from about 45°C to about 80°C (although temperatures outside these ranges may be utilized), which may be below the glass transition temperature of the resin as discussed above.

Following aggregation to the desired particle size, the particles may then be coalesced to the desired final shape, the coalescence being achieved by, for example, heating the mixture to a temperature of from about 65°C to about 105°C, in embodiments from about 70°C to about 95°C (although temperatures outside these ranges may be utilized), which may be at or above the glass transition temperature of the resin, and/or increasing the stirring, for example to from about 400 rpm to about 1,000 rpm, in embodiments from about 500 rpm to about 800 rpm, although speeds outside these ranges may be utilized. Higher or lower temperatures may be used, it being understood that the temperature is a function of the resins used for the binder. Coalescence may be accomplished over a period of from about 0.1 to about 9 hours, in embodiments from about 0.5 to about 4 hours, although times outside these ranges may be utilized.

After aggregation and/or coalescence, the mixture may be cooled to room temperature, such as from about 20°C to about 25°C. The cooling may be rapid or slow, as desired. A suitable cooling method may include introducing cold water to a jacket around the reactor. After cooling, the toner particles may be optionally washed with water, and then dried. Drying may be accomplished by any suitable method for drying including, for example, freeze-drying.

### Finishing

After aggregation, but prior to coalescence, once the desired final size of the toner particles is achieved, the pH of the mixture may be adjusted with a base to a value of from about 3 to about 10, and in embodiments from about 5 to about 9, although pH outside these ranges may be utilized. The adjustment of the pH may be utilized to freeze, that is to stop, toner growth. The base utilized to stop toner growth may include any suitable base such as, for example, alkali metal hydroxides such as, for example, sodium hydroxide, potassium hydroxide, ammonium hydroxide, combinations thereof, and the like. In embodiments, ethylene diamine tetraacetic acid (EDTA) may be added to help adjust the pH to the desired values noted above.

### Additives

In embodiments, the toner particles may also contain other optional additives, as desired or required. For example, the toner may include positive or negative charge control agents, for example in an amount of from about 0.1 to about 10 percent by weight of the toner, in embodiments from about 1 to about 3 percent by weight of the toner, although amounts outside these ranges may be utilized. Examples of suitable charge control agents include quaternary ammonium compounds inclusive of alkyl pyridinium halides; bisulfates; alkyl pyridinium compounds, including those disclosed in U.S. Patent No. 4,298,672; organic sulfate and sulfonate compositions, including those disclosed in U.S. Patent No. 4,338,390; cetyl pyridinium tetrafluoroborates; distearyl dimethyl ammonium methyl sulfate; aluminum salts such as BONTRON E84™ or E88™ (Hodogaya Chemical); combinations thereof, and the like. Such charge control agents may be applied simultaneously with the shell resin described above or after application of the shell resin.

There can also be blended with the toner particles external additive particles including flow aid additives, which additives may be present on the surface of the toner particles. Examples of these additives include metal oxides such as titanium oxide, silicon oxide, tin oxide, mixtures thereof, and the like; colloidal and amorphous silicas, such as AEROSIL®, metal salts and metal salts of fatty acids inclusive of zinc stearate, aluminum oxides, cerium oxides, and mixtures thereof. Each of these external additives may be present in an amount of from about 0.1 percent by weight to about 5 percent by weight of the toner, in embodiments of from about 0.25 percent by weight to about 3 percent by weight of the toner, although amounts outside these ranges may be utilized. Suitable additives include those disclosed in U.S. Patent Nos. 3,590,000, 3,800,588, and 6,214,507.

In embodiments, toners of the present disclosure may be utilized as low-melt polyester toners. In embodiments, the dry toner particles, exclusive of external surface additives, may have the following characteristics:

(1) Volume average diameter (also referred to as "volume average particle diameter") of from about 3 to about 25 µm, in embodiments from about 4 to about 15 µm, in other embodiments from about 5 to about 12 µm, although values outside these ranges may be obtained.

(2) Number Average Geometric Size Distribution (GSDn) and/or Volume Average Geometric Size Distribution (GSDv) of from about 1.05 to about 1.55, in embodiments from about 1.1 to about 1.4, although values outside these ranges may be obtained.

(3) Circularity of from about 0.9 to about 0.99, although values outside these ranges may be obtained (measured with, for example, a Sysmex FPIA 2100 analyzer).

The characteristics of the toner particles may be determined by any suitable technique and apparatus. Volume average particle diameter D₅₀ᵥ, GSDv, and GSDn may be measured by means of a measuring instrument such as a Beckman Coulter Multisizer 3, operated in accordance with the manufacturer's instructions. Representative sampling may occur as follows: a small amount of toner sample, about 1 gram, may be obtained and dispersed in about 200 ml of water, filtered through a 25 micrometer screen, then put in isotonic solution to obtain a concentration of about 10%, with the sample then run in a Beckman Coulter Multisizer 3.

Toners produced in accordance with the present disclosure may possess excellent charging characteristics when exposed to extreme relative humidity (RH) conditions. The low-humidity zone (C zone) is about 10°C/15% RH, while the high humidity zone (A zone) is about 28°C/85% RH. Toners of the present disclosure may also possess a parent toner charge per mass ratio (Q/M) of from about -3 µC/g to about -35 µC/g, and a final toner charging after surface additive blending of from -5 µC/g to about -50 µC/g, although values outside these ranges may be obtained.

In accordance with the present disclosure, the charging of the toner particles may be enhanced, so less surface additives may be required, and the final toner charging may thus be higher to meet machine charging requirements.

### Uses

The polymerization synthesis according to the present disclosure may be used to prepare resins for use in subsequent synthesis of emulsion aggregation toners either in the presence or absence of solvents. Copolymers possessing both crystalline and amorphous blocks may be produced. The disclosed synthesis also provides for reduced reaction times and energy costs, since a single copolymer may be utilized in the production of toners, instead of separate crystalline polyesters and amorphous polyesters.

In addition, lactones suitable for polymerization may be readily available, making the starting materials relatively inexpensive since the lactones are widely used in other industries, for example as fragrances, food additives, and the like.

### Developers

The toner particles may be formulated into a developer composition. The toner particles may be mixed with carrier particles to achieve a two-component developer composition. The toner concentration in the developer may be from about 1% to about 25% by weight of the total weight of the developer, in embodiments from about 2% to about 15% by weight of the total weight of the developer, although amounts outside these ranges may be utilized.

### Carriers

Examples of carrier particles that can be utilized for mixing with the toner include those particles that are capable oftriboelectrically obtaining a charge of opposite polarity to that of the toner particles. Illustrative examples of suitable carrier particles include granular zircon, granular silicon, glass, steel, nickel, ferrites, iron ferrites, silicon dioxide, and the like. Other carriers include those disclosed in U.S. Patent Nos. 3,847,604, 4,937,166, and 4,935,326.

The selected carrier particles can be used with or without a coating. In embodiments, the carrier particles may include a core with a coating thereover which may be formed from a mixture of polymers that are not in close proximity thereto in the triboelectric series. The coating may include fluoropolymers, such as polyvinylidene fluoride resins, terpolymers of styrene, methyl methacrylate, and/or silanes, such as triethoxy silane, tetrafluoroethylenes, other known coatings and the like. For example, coatings containing polyvinylidenefluoride, available, for example, as KYNAR 301F™, and/or polymethylmethacrylate, for example having a weight average molecular weight of about 300,000 to about 350,000, such as commercially available from Soken, may be used. In embodiments, polyvinylidenefluoride and polymethylmethacrylate (PMMA) may be mixed in proportions of from about 30 to about 70 weight % to about 70 to about 30 weight %, in embodiments from about 40 to about 60 weight % to about 60 to about 40 weight %, although amounts outside these ranges may be utilized. The coating may have a coating weight of, for example, from about 0.1 to about 5% by weight of the carrier, in embodiments from about 0.5 to about 2% by weight of the carrier, although amounts outside these ranges may be utilized.

In embodiments, PMMA may optionally be copolymerized with any desired comonomer, so long as the resulting copolymer retains a suitable particle size. Suitable comonomers can include monoalkyl, or dialkyl amines, such as a dimethylaminoethyl methacrylate, diethylaminoethyl methacrylate, diisopropylaminoethyl methacrylate, or t-butylaminoethyl methacrylate, and the like. The carrier particles may be prepared by mixing the carrier core with polymer in an amount from about 0.05 to about 10 percent by weight, in embodiments from about 0.01 percent to about 3 percent by weight (although amounts outside these ranges may be utilized), based on the weight of the coated carrier particles, until adherence thereof to the carrier core by mechanical impaction and/or electrostatic attraction.

Various effective suitable means can be used to apply the polymer to the surface of the carrier core particles, for example, cascade roll mixing, tumbling, milling, shaking, electrostatic powder cloud spraying, fluidized bed, electrostatic disc processing, electrostatic curtain, combinations thereof, and the like. The mixture of carrier core particles and polymer may then be heated to enable the polymer to melt and fuse to the carrier core particles. The coated carrier particles may then be cooled and thereafter classified to a desired particle size.

In embodiments, suitable carriers may include a steel core, for example of from about 25 to about 100 µm in size, in embodiments from about 50 to about 75 µm in size (although sizes outside these ranges may be utilized), coated with about 0.5% to about 10% by weight, in embodiments from about 0.7% to about 5% by weight (although amounts outside these ranges may be utilized), of a conductive polymer mixture including, for example, methylacrylate and carbon black using the process described in U.S. Patent Nos. 5,236,629 and 5,330,874.

The carrier particles can be mixed with the toner particles in various suitable combinations. The concentrations may be from about 1% to about 20% by weight of the toner composition. However, different toner and carrier percentages may be used to achieve a developer composition with desired characteristics.

### Imaging

The toners can be utilized for electrophotographic or xerographic processes, including those disclosed in U.S. Patent No. 4,295,990. In embodiments, any known type of image development system may be used in an image developing device, including, for example, magnetic brush development, jumping single-component development, hybrid scavengeless development (HSD), and the like. These and similar development systems are within the purview of those skilled in the art.

Imaging processes include, for example, preparing an image with a xerographic device including a charging component, an imaging component, a photoconductive component, a developing component, a transfer component, and a fusing component. In embodiments, the development component may include a developer prepared by mixing a carrier with a toner composition described herein. The xerographic device may include a high speed printer, a black and white high speed printer, a color printer, and the like.

Once the image is formed with toners/developers via a suitable image development method such as any one of the aforementioned methods, the image may then be transferred to an image receiving medium such as paper and the like. In embodiments, the toners may be used in developing an image in an image-developing device utilizing a fuser roll member. Fuser roll members are contact fusing devices that are within the purview of those skilled in the art, in which heat and pressure from the roll may be used to fuse the toner to the image-receiving medium. In embodiments, the fuser member may be heated to a temperature above the fusing temperature of the toner, for example to temperatures of from about 70°C to about 160°C, in embodiments from about 80°C to about 150°C, in other embodiments from about 90°C to about 140°C (although temperatures outside these ranges may be utilized), after or during melting onto the image receiving substrate.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" refers to a temperature of from about 20 ° C to about 25° C.

### EXAMPLES

### EXAMPLE 1

Synthesis of poly(caprolactone-block-lactide). About 20 parts caprolactone is dissolved in about 200 parts toluene under a nitrogen atmosphere at room temperature. Aluminum isopropoxide (1 part) is added and the reaction mixture is stirred until no unreacted caprolactone remains in the reaction mixture. A solution of 80 parts D,L-lactide in about 200 parts toluene is then added to the reaction mixture, which is stirred until no unreacted D,L-lactide remains. Following this, a solution of 2 parts sulfonated 4-phenylcaprolactone in 10 parts toluene is added to the reaction mixture, which is stirred until no unreacted sulfonated 4-phenylcaprolactone remains. The reaction mixture is added to methanol to isolate a block co-polymer product, which is washed with methanol.

## Claims

1. A process comprising:
contacting a first component with a catalyst, optionally in solution;
polymerizing the first component to form a first block of a copolyester resin;
contacting the first block, optionally in solution, with a second component;
polymerizing the second component to form a second block of the copolyester resin linked to the first block; and
recovering the copolyester resin comprising a crystalline block and an amorphous block;
wherein one of the first and second components is at least one precursor of an amorphous block comprising a lactone, a cyclic anhydrid, a cyclic carbonateor a combination thereof, and the other of the first and second components is at least one precursor of a crystalline block comprising a lactone, a cyclic anhydrid, a cyclic carbonate or a combination thereof

2. A process as in claim 1, wherein
the first component is at least one precursor of an amorphous block comprising a lactone selected from the group consisting of 4-tert-butylcaprolactone, 4-phenylcaprolactone, 4-methylcaprolactone, 3,5-dimethylcaprolactone, 3-isochromanone, 4-(sulfonatophenyl)caprolactone, lactide, glycolide, cyclic anhydrides, cyclic carbonates and combinations thereof,
the polymerizing the at least one precursor of the amorphous block forms an amorphous block;
the second component is at least one precursor of a crystalline block comprising a lactone selected from the group consisting of caprolactone, 1,4-dioxan-2-one, valerolactone and combinations thereof, optionally with a catalyst, optionally in solution;
the polymerizing of the at least one precursor of the crystalline block to form a crystalline block linked to the amorphous block.

3. A process as in claim 1, wherein either the first component or the second component comprises a precursor of the crystalline block selected from the group consisting of lactones, cyclic anhydrides, cyclic carbonates, and combinations thereof, and the other component comprises a precursor of the amorphous block comprising a lactone selected from the group consisting of 4-tert-butylcaprolactone, 4-phenylcaprolactone, 3-isochromanone, 4-methylcaprolactone, 3,5-dimethylcaprolactone, 4-(sulfonatophenyl)caprolactone, lactide, glycolide, cyclic anhydrides, cyclic carbonates, and combinations thereof.

4. A process as in claim 3, wherein the precursor of the crystalline block comprises a lactone selected from the group consisting of caprolactone, 1,4-dioxan-2-one, valerolactone, and combinations thereof, a cyclic anhydride selected from the group consisting of succinic anhydride, glutaric anhydride, maleic anhydride, cyclopentane-1,2-dicarboxylic anhydride, cyclohexene-1,2-dicarboxylic anhydride, 3-bicyclo[2,2,2]octadicarboxylic anhydride, phthalic anhydride, naphthalene-dicarboxylic anhydride, pyridine-dicarboxylic anhydride, thiophene-dicarboxylic anhydride, and combinations thereof, a cyclic carbonate selected from the group consisting of trimethylene carbonate, carboxytrimethylene carbonate, esters of carboxytrimethylene carbonate, and combinations thereof

5. A process as in claim 1 or 2, wherein the catalyst is selected from the group consisting of aluminum isopropoxide, yttrium isopropoxide, stannous octoate, scandium trifluoromethane sulfonate, stannous trifluoromethane sulfonate, and combinations thereof.

6. A process as in claim 1, wherein the catalyst comprises a lipase obtained from an organism selected from the group consisting of Pseudomonas aeruginosa, Pseudomonas cepacia, Pseudomonas fluorescens, Aspegillus niger, Candida antarctica, Candida cylindracea, Klebsiella oxytota, and Mucor meihei; preferably
the lipase is selected from the group consisting of lipase PA, lipase PC, lipase PF, lipase A, lipase CA, lipase B, lipase CC, lipase K, lipase MM, and combinations thereof.

7. A process as in claim 1 or 2, wherein the optional solution comprises a solvent selected from the group consisting of water, toluene, benzene, xylene, tetrahydrofuran, dichloromethane, and combinations thereof.

8. A process as in claim 1 or 2, wherein the crystalline block of the copolyester resin is present in an amount of from 1 to 90 percent by weight of the copolyester resin and possesses a melting temperature of from 20°C to 200°C, and the amorphous block of the copolyester resin is present in an amount of from 10 to 99 percent by weight of the copolyester resin and possesses a glass transition temperature of from 0°C to 200°C.

9. A process as in claim 8, further comprising contacting the copolyester resin with water to form core-shell particles, the core-shell particles comprising the crystalline block as the core and the amorphous block as the shell; preferably
introducing cross-linkable vinylic groups at an end of the crystalline block, the amorphous block, or both, and subjecting the vinylic groups to cross-linking.

10. A process as in claim 9, further comprising:
contacting the core-shell particles with at least one colorant, an optional wax, and an optional surfactant to form toner particles; and recovering the toner particles.

11. A process as in claim 2, wherein the copolyester resin comprises poly[caprolactone-block-4-phenylcaprolactone-block-4-(sulfonatophenyl)caprolactone], and the core-shell particles comprise an inner core of polycaprolactone, an intermediate layer comprising poly(4-phenylcaprolactone), and a shell comprising poly(4-(sulfonatophenyl)caprolactone).

12. A composition comprising:
a copolyester resin comprising core-shell particles comprising a crystalline block obtained from the polymerization of a lactone selected from the group consisting of caprolactone, 1,4-dioxan-2-one, valerolactone, and combinations thereof as the core, in combination with an amorphous block obtained from the polymerization of a lactone selected from the group consisting of 4-tert-butylcaprolactone, 4-phenylcaprolactone, 4-methylcaprolactone, 3,5-dimethylcaprolactone, 3-isochromanone, lactide, glycolide, 4-(sulfonatophenyl)caprolactone, and combinations thereof as the shell;
at least one colorant; and
an optional wax, and an optional surfactant,
wherein the crystalline block of the copolyester resin is present in an amount of from 1 to about 90 percent by weight of the copolyester resin and possesses a melting temperature of from20°C to 200°C, and the amorphous block of the copolyester resin is present in an amount of from 10 to 99 percent by weight of the copolyester resin and possesses a glass transition temperature of from 0°C to 200°C.

13. A composition as in claim 12, wherein the copolyester resin further comprises a cross-linkable vinylic group at an end of the crystalline block, the amorphous block, or both.

## Patentansprüche

1. Verfahren umfassend:
das Inkontaktbringen einer ersten Komponente mit einem Katalysator, gegebenenfalls in Lösung;
das Polymerisieren der ersten Komponente, um einen ersten Block eines Copolyesterharzes zu bilden;
das Inkontaktbringen des ersten Blocks, gegebenenfalls in Lösung, mit einer zweiten Komponente;
das Polymerisieren der zweiten Komponente, um einen zweiten Block des Copolyesterharzes zu bilden, der mit dem ersten Block verknüpft ist; und
das Gewinnen des Copolyesterharzes, das einen kristallinen Block und einen amorphen Block umfasst;
wobei eine von den ersten und zweiten Komponenten wenigstens eine Vorstufe eines amorphen Blocks ist, umfassend ein Lacton, ein cyclisches Anhydrid, ein cyclisches Carbonat oder eine Kombination davon, und die andere von den ersten und zweiten Komponenten wenigstens eine Vorstufe eines kristallinen Blocks ist, umfassend ein Lacton, ein cyclisches Anhydrid, ein cyclisches Carbonat oder eine Kombination davon.

2. Verfahren nach Anspruch 1, wobei
die erste Komponente wenigstens eine Vorstufe eines amorphen Blocks ist, umfassend ein Lacton, ausgewählt aus der Gruppe bestehend aus 4-tert-Butylcaprolacton, 4-Phenylcaprolacton, 4-Methylcaprolacton, 3,5-Dimethylcaprolacton, 3-Isochromanon, 4-(Sulfonatophenyl)caprolacton, Lactid, Glycolid, cyclischen Anhydriden, cyclischen Carbonaten und Kombinationen davon,
das Polymerisieren der wenigstens einen Vorstufe des amorphen Blocks einen amorphen Block bildet;
die zweite Komponente wenigstens eine Vorstufe eines kristallinen Blocks ist, umfassend ein Lacton, ausgewählt aus der Gruppe bestehend aus Caprolacton, 1,4-Dioxan-2-on, Valerolacton und Kombinationen davon, gegebenenfalls mit einem Katalysator, gegebenenfalls in Lösung;
das Polymerisieren der wenigstens einen Vorstufe des kristallinen Blocks, um einen kristallinen Block zu bilden, der mit dem amorphen Block verknüpft ist.

3. Verfahren nach Anspruch 1, wobei entweder die erste Komponente oder die zweite Komponente eine Vorstufe des kristallinen Blocks, ausgewählt aus der Gruppe bestehend aus Lactonen, cyclischen Anhydriden, cyclischen Carbonaten und Kombinationen davon, umfasst, und die andere Komponente eine Vorstufe des amorphen Blocks, umfassend ein Lacton, ausgewählt aus der Gruppe bestehend aus 4-tert-Butylcaprolacton, 4-Phenylcaprolacton, 3-Isochromanon, 4-Methylcaprolacton, 3,5-Dimethylcaprolacton, 4-(Sulfonatophenyl)caprolacton, Lactid, Glycolid, cyclischen Anhydriden, cyclischen Carbonaten und Kombinationen davon, umfasst.

4. Verfahren nach Anspruch 3, wobei die Vorstufe des kristallinen Blocks ein Lacton, ausgewählt aus der Gruppe bestehend aus Caprolacton, 1,4-Dioxan-2-on, Valerolacton und Kombinationen davon, ein cyclisches Anhydrid, ausgewählt aus der Gruppe bestehend aus Bernsteinsäureanhydrid, Glutarsäureanhydrid, Maleinsäureanhydrid, Cyclopentan-1,2-dicarbonsäureanhydrid, Cyclohexen-1,2-dicarbonsäureanhydrid, 3-Bicyclo[2.2.2]octa-dicarbonsäureanhydrid, Phthalsäureanhydrid, Naphthalin-dicarbonsäureanhydrid, Pyridindicarbonsäureanhydrid, Thiophen-dicarbonsäureanhydrid und Kombinationen davon, ein cyclisches Carbonat, ausgewählt aus der Gruppe bestehend aus Trimethylencarbonat, Carboxytrimethylencarbonat, Estern von Carboxytrimethylencarbonat und Kombinationen davon, umfasst.

5. Verfahren nach Anspruch 1 oder 2, wobei der Katalysator ausgewählt ist aus der Gruppe bestehend aus Aluminiumisopropoxid, Yttriumisopropoxid, Zinn(II)-octoat, Scandiumtrifluormethansulfonat, Zinn(II)-trifluormethansulfonat und Kombinationen davon.

6. Verfahren nach Anspruch 1, wobei der Katalysator eine Lipase umfasst, die aus einem Organismus erhalten wird, der ausgewählt ist aus der Gruppe bestehend aus Pseudomonas aeruginosa, Pseudomonas cepacia, Pseudomonas fluorescens, Aspergillus niger, Candida antarctica, Candida cylindracea, Klebsiella oxytoca und Mucor meihei; vorzugsweise
die Lipase ausgewählt ist aus der Gruppe bestehend aus Lipase PA, Lipase PC, Lipase PF, Lipase A, Lipase CA, Lipase B, Lipase CC, Lipase K, Lipase MM und Kombinationen davon.

7. Verfahren nach Anspruch 1 oder 2, wobei die optionale Lösung ein Lösungsmittel umfasst, das ausgewählt ist aus der Gruppe bestehend aus Wasser, Toluol, Benzol, Xylol, Tetrahydrofuran, Dichlormethan und Kombinationen davon.

8. Verfahren nach Anspruch 1 oder 2, wobei der kristalline Block des Copolyesterharzes in einer Menge von 1 bis 90 Gew.-% des Copolyesterharzes vorhanden ist und eine Schmelztemperatur von 20°C bis 200°C besitzt, und der amorphe Block des Copolyesterharzes in einer Menge von 10 bis 99 Gew.-% des Copolyesterharzes vorhanden ist und eine Glasübergangstemperatur von 0°C bis 200°C besitzt.

9. Verfahren nach Anspruch 8, außerdem umfassend das Inkontaktbringen des Copolyesterharzes mit Wasser, um Kern-Hülle-Teilchen zu bilden, wobei die Kern-Hülle-Teilchen den kristallinen Block als den Kern und den amorphen Block als die Hülle umfassen; vorzugsweise
das Einführen von vernetzbaren Vinylgruppen an einem Ende von dem kristallinen Block, dem amorphen Block oder von beiden, und das Unterwerfen der Vinylgruppen einer Vernetzung.

10. Verfahren nach Anspruch 9, außerdem umfassend:
das Inkontaktbringen der Kern-Hülle-Teilchen mit wenigstens einem Farbmittel, einem optionalen Wachs und einem optionalen oberflächenaktiven Stoff, um Tonerteilchen zu bilden; und das Gewinnen der Tonerteilchen.

11. Verfahren nach Anspruch 2, wobei das Copolyesterharz Poly[caprolacton-block-4-phenylcaprolacton-block-4-(sulfonatophenyl)caprolacton] umfasst und die Kern-Hülle-Teilchen einen inneren Kern aus Polycaprolacton, eine Zwischenschicht, umfassend Poly(4-phenylcaprolacton), und eine Hülle, umfassend Poly(4-(sulfonatophenyl)caprolacton), umfassen.

12. Zusammensetzung umfassend:
ein Copolyesterharz umfassend Kern-Hülle-Teilchen umfassend einen kristallinen Block, der durch die Polymerisation eines Lactons, ausgewählt aus der Gruppe bestehend aus Caprolacton, 1,4-Dioxan-2-on, Valerolacton und Kombinationen davon, erhalten wird, als den Kern, in Kombination mit einem amorphen Block, der durch die Polymerisation eines Lactons, ausgewählt aus der Gruppe bestehend aus 4-tert-Butylcaprolacton, 4-Phenylcaprolacton, 4-Methylcaprolacton, 3,5-Dimethylcaprolacton, 3-Isochromanon, Lactid, Glycolid, 4-(Sulfonatophenyl)caprolacton und Kombinationen davon, erhalten wird, als die Hülle;
wenigstens ein Farbmittel; und
ein optionales Wachs und einen optionalen oberflächenaktiven Stoff,
wobei der kristalline Block des Copolyesterharzes in einer Menge von 1 bis ungefähr 90 Gew.-% des Copolyesterharzes vorhanden ist und eine Schmelztemperatur von 20°C bis 200°C besitzt, und der amorphe Block des Copolyesterharzes in einer Menge von 10 bis 99 Gew.-% des Copolyesterharzes vorhanden ist und eine Glasübergangstemperatur von 0°C bis 200°C besitzt.

13. Zusammensetzung nach Anspruch 12, wobei das Copolyesterharz außerdem eine vernetzbare Vinylgruppe an einem Ende von dem kristallinen Block, dem amorphen Block oder von beiden umfasst.

## Revendications

1. Processus comprenant les étapes consistant à :
mettre en contact un premier composant avec un catalyseur, facultativement en solution ;
polymériser le premier composant pour former un premier bloc d'une résine copolyester ;
mettre en contact le premier bloc, facultativement en solution, avec un deuxième composant ;
polymériser le deuxième composant pour former un deuxième bloc de résine copolyester liée au premier bloc ; et
récupérer la résine copolyester comprenant un bloc cristallin et un bloc amorphe ;
dans lequel l'un des premier et deuxième composants est au moins un précurseur d'un bloc amorphe comprenant une lactone, un anhydride cyclique, un carbonate cyclique ou une combinaison de ceux-ci, et l'autre des premier et deuxième composants est au moins un précurseur d'un bloc cristallin comprenant une lactone, un anhydride cyclique, un carbonate cyclique ou une combinaison de ceux-ci.

2. Processus selon la revendication 1, dans lequel
le premier composant est au moins un précurseur d'un bloc amorphe comprenant une lactone choisie parmi le groupe constitué de 4-tert-butylcaprolactone, de 4-phenylcaprolactone, de 4-méthylcaprolactone, de 3,5-diméthylcaprolactone, de 3-isochromanone, de 4 - (sulfonatophényl) caprolactone, de lactide, de glycolide, des anhydrides cycliques, des carbonates cycliques et des combinaisons de ceux-ci,
la polymérisation de l'au moins un précurseur du bloc amorphe forme un bloc amorphe ;
le deuxième composant est au moins un précurseur d'un bloc cristallin comprenant une lactone choisie parmi le groupe constitué de caprolactone, de 1,4-dioxan-2-one, de valérolactone et des combinaisons de celles-ci, facultativement avec un catalyseur, facultativement en solution ;
la polymérisation de l'au moins un précurseur du bloc cristallin pour former un bloc cristallin lié au bloc amorphe.

3. Processus selon la revendication 1, dans lequel soit le premier composant ou le deuxième composant comprend un précurseur du bloc cristallin choisi parmi le groupe constitué de lactones, des anhydrides cycliques, des carbonates cycliques, et des combinaisons de ceux-ci, et l'autre composant comprend un précurseur du bloc amorphe comprenant une lactone choisie parmi le groupe constitué de 4-tert--butylcaprolactone, de 4-phenylcaprolactone, de 3-isochromanone, de 4-methylcaprolactone, de 3,5-diméthylcaprolactone, de 4 - (sulfonatophényl) caprolactone, de lactide, de glycolide, des anhydrides cycliques, des carbonates cycliques, et des combinaisons de ceux-ci.

4. Processus selon la revendication 3, dans lequel le précurseur du bloc cristallin comprend une lactone choisie parmi le groupe constitué de caprolactone, de 1,4-dioxan-2-one, de valérolactone, et des combinaisons de celles-ci, d'un anhydride cyclique choisi parmi le groupe constitué d'anhydride succinique, d'anhydride glutarique, d'anhydride maléique, d'anhydride cyclopentane-1,2-dicarboxylique, d'anhydride cyclohexène-1,2-dicarboxylique, d'anhydride 3-bicyclo[2,2,2]octadicarboxylique, d'anhydride phtalique, d'anhydride naphtalène-dicarboxylique, d'anhydride pyridine-dicarboxylique, d'anhydride thiophène-dicarboxylique, et des combinaisons de ceux-ci, un carbonate cyclique choisi parmi le groupe constitué de carbonate de triméthylène, de carbonate de carboxytriméthylène, des esters de carbonate de carboxytriméthylène, et des combinaisons de ceux-ci.

5. Processus selon la revendication 1 ou 2, dans lequel le catalyseur est choisi parmi le groupe constitué d'isopropoxyde d'aluminium, d'isopropoxyde d'yttrium, d'octoate stanneux, de trifluorométhanesulfonate de scandium, de trifluorométhanesulfonate d'étain, et des combinaisons de ceux-ci.

6. Processus selon la revendication 1, dans lequel le catalyseur comprend une lipase obtenue à partir d'un organisme choisi parmi le groupe constitué de Pseudomonas aeruginosa, de Pseudomonas cepacia, de Pseudomonas fluorescens, d'Aspegillus niger, de Candida antarctica, de Candida cylindracea, de Klebsiella oxytoca, et de Mucor meihei ; de préférence
la lipase est choisie parmi le groupe constitué de lipase PA, de lipase PC, de lipase PF, de lipase A, de lipase CA, de lipase B, de lipase CC, de lipase K, de lipase MM, et des combinaisons de celles-ci.

7. Processus selon la revendication 1 ou 2, dans lequel la solution facultative comprend un solvant choisi parmi le groupe constitué de l'eau, de toluène, de benzène, de xylène, de tétrahydrofuranne, de dichlorométhane, et des combinaisons de ceux-ci.

8. Processus selon la revendication 1 ou 2, dans lequel le bloc cristallin de la résine copolyester est présent en une quantité de 1 à 90 pour cent en poids de la résine copolyester et possède une température de fusion de 20°C à 200°C, et le bloc amorphe de la résine copolyester est présent en une quantité de 10 à 99 pour cent en poids de la résine copolyester et possède une température de transition vitreuse de 0°C à 200°C.

9. Processus selon la revendication 8, comprenant en outre les étapes consistant à mettre en contact la résine copolyester avec de l'eau pour former des particules coeur-enveloppe, les particules coeur-enveloppe comprenant le bloc cristallin comme étant le coeur et le bloc amorphe comme étant l'enveloppe ; de préférence
à introduire des groupes vinyliques réticulables à une extrémité du bloc cristallin, du bloc amorphe, ou des deux, et à soumettre les groupes vinyliques à la réticulation.

10. Processus selon la revendication 9, comprenant en outre les étapes consistant :
à mettre en contact des particules coeur-enveloppe avec au moins un colorant, une cire facultative, et un tensioactif facultatif pour former des particules d'encre ; et à récupérer les particules d'encre.

11. Processus selon la revendication 2, dans lequel la résine copolyester comprend du poly [caprolactone-bloc-4-phenylcaprolactone-bloc-4-(sulfonatophényl) caprolactone], et les particules coeur-enveloppe comprennent un coeur intérieur de la polycaprolactone, une couche intermédiaire comprenant du poly (4-phenylcaprolactone), et une enveloppe comprenant un poly (4 - (sulfonatophényl) caprolactone).

12. Composition comprenant:
une résine de copolyester comprenant des particules coeur-enveloppe comprenant un bloc cristallin obtenu de la polymérisation d'une lactone choisie parmi le groupe constitué de caprolactone, de 1,4-dioxan-2-one, de valérolactone, et des combinaisons de celles-ci comme étant le coeur, en combinaison avec un bloc amorphe obtenu de la polymérisation d'une lactone choisie parmi le groupe constitué de 4-tert-butylcaprolactone, de 4-phenylcaprolactone, de 4-methylcaprolactone, de 3,5-diméthylcaprolactone, de 3-isochromanone, de lactide, de glycolide, de 4 - (sulfonatophényl) caprolactone, et des combinaisons de ceux-ci comme étant l'enveloppe ;
au moins un colorant ; et
une cire facultative, et un tensioactif facultatif,
dans lequel le bloc cristallin de la résine copolyester est présent en une quantité de 1 à environ 90 pour cent en poids de la résine copolyester et possède une température de fusion de 20°C à 200°C, et le bloc amorphe de la résine copolyester est présent en une quantité de 10 à 99 pour cent en poids de la résine copolyester et possède une température de transition vitreuse de 0°C à 200°C.

13. Composition selon la revendication 12, dans laquelle la résine copolyester comprend en outre un groupe vinylique réticulable à une extrémité du bloc cristallin, du bloc amorphe, ou des deux.
